(19) Europäisches Patentamt
European Patent Office
Office européen des brevets

(11) **EP 4 383 189 A1**

(12) **EUROPEAN PATENT APPLICATION**

(43) Date of publication:
**12.06.2024 Bulletin 2024/24**

(21) Application number: **22212260.8**

(22) Date of filing: **08.12.2022**

(51) International Patent Classification (IPC):
**G06T 7/11** (2017.01)   **G06T 7/187** (2017.01)

(52) Cooperative Patent Classification (CPC):
**G06T 7/11; G06T 7/187;** G06T 2207/10081;
G06T 2207/10088; G06T 2207/10096;
G06T 2207/20041; G06T 2207/20101;
G06T 2207/30048; G06T 2207/30104

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WIEMKER, Rafael
Eindhoven (NL)**

• **NICKISCH, Hannes**
**Eindhoven (NL)**
• **BONTUS, Claas**
**5656AG Eindhoven (NL)**
• **BROSCH, Tom**
**5656AG Eindhoven (NL)**
• **PETERS, Jochen**
**Eindhoven (NL)**
• **WEESE, Rolf Jürgen**
**Eindhoven (NL)**

(74) Representative: **Philips Intellectual Property &
Standards
High Tech Campus 52
5656 AG Eindhoven (NL)**

(54) **APPARATUS FOR ASSISTING IN DETERMINING A FUNCTIONAL SUBDIVISION INTO ARTERIAL FEEDER REGIONS IN AN ORGAN OF A SUBJECT**

(57)    It is an object of the invention to provide an apparatus 110 that allows for assisting a user in determining an improved subdivision into arterial feeding regions in an organ of a patient 121. An image providing unit 111 provides an image of an organ of a subject. A geodesic distance map generation unit 113 generates a geodesic distance map for a region of interest based on the anatomical image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image. A geodesic distance map providing unit 114 provides the geodesic distance map to a user and/or to further processing to determine the arterial feeding regions.

FIG. 1

EP 4 383 189 A1

**Description**

FIELD OF THE INVENTION

The invention relates to an apparatus, a system comprising the apparatus, a method and a computer program product for assisting in determining a functional subdivision into arterial feeding regions in an organ of a subject.

BACKGROUND OF THE INVENTION

The determination of arterial feeding regions in an organ of a subject is important for the diagnosis and treatment of many medical conditions, in particular, for the diagnosis and treatment of conditions of the heart muscle. Generally, medical images acquired under the influence of contrast agents are utilized for determining the perfusion in different tissue regions of the organ and thus also the feeding of these regions from the respective arteries. In order to determine the different feeding regions of an artery based on the perfusion, it is known to utilize schematic, geometrically predetermined feeding region atlases for a respective organ. This leads to a generally rigid mapping of the feeding regions on the organ without utilizing the information provided by the functional image, for instance, the contrast agent uptake and perfusion when determining the feeding regions. Thus, it would be advantageous if a medical practitioner can be assisted in determining the arterial feeding regions of an organ for each individual patient.

SUMMARY OF THE INVENTION

It is an object of the present invention to provide an apparatus, a system comprising the apparatus, a method and a computer program product that allow for assisting a user in determining an improved, in particular, individual functional subdivision into arterial feeding regions in an organ of a patient.
In a first aspect of the invention, an apparatus for assisting in determining a functional subdivision into arterial feeding regions in an organ of a subject, wherein the apparatus comprises a) an image providing unit for providing an image of an organ of a subject, wherein the image is an anatomical image acquired after the administration of a contrast agent, b) a region of interest defining unit for defining a region of interest in the image for which the arterial feeding regions are to be determined, c) a geodesic distance map generation unit for generating a geodesic distance map for the region of interest based on the anatomical image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image, d) a geodesic distance map providing unit for providing the geodesic distance map to a user and/or to further processing to determine the arterial feeding regions.
Since a geodesic distance map is generated for the volume of interest based on the image, wherein a voxel of the geodesic distance map is given by a cumulated path cost from a predetermined seed to a respective voxel and wherein the path cost is determined based on a cost function taking into account a vesselness filter response and/or a contrast agent density determined, respectively, based on the image, the information on the feeding of a certain area of tissue is clearly shown on the geodesic distance map. Moreover, since the geodesic distance map is generated based on an image of the respective volume of interest of a patient, the resulting determination of arterial feeding regions can be adapted individually to the respective patient. Thus, the apparatus allows for an improved assistance of a user when determining a functional subdivision into arterial feeding regions in an organ of a subject, in particular, in order to individualize the determined arterial feeding regions.
The apparatus is configured for assisting in determining a functional subdivision into arterial feeding regions in an organ of a subject. The apparatus can be realized in form of hardware and/or software provided by a general or dedicated computer system. In particular, the apparatus can also be realized in distributed computing, for example, can be realized as part of a computer network in which the functions of the apparatus are provided by different processors, servers or computer systems that can be located at the same or at different locations. The subject can be a human being or an animal and in the following can also be refer to as a patient. The organ for which the arterial feeding regions are to be determined can be any organ of a person or animal but is preferably a heart of the subject.
The image providing unit is configured for providing an image of an organ of the subject. In particular, the image providing unit can refer to a storage unit or can be communicatively coupled to a storage unit, wherein the image is already stored on the storage unit and the image providing unit is configured for providing the image stored on the storage unit. Further, the image providing unit can also be a receiving unit for receiving the image, for instance, from an input unit or via an interface directly from the image acquisition unit, and for providing the received image. Moreover, the image providing unit can also be an image acquisition unit, for instance, a CT or MRI unit configured for acquiring the image and further configured for providing the same. The image is an anatomical image acquired after the administration of a contrast agent such that the image provides information on the transport and perfusion of a fluid in the vessel system, in particular, in the arterial system, of the patient. Preferably, the anatomical image is an anatomical volume image, i.e. a 3D anatomical image. This is in particular suitable if the organ is the heart of a patient. However, in cases in which the organ is naturally flat or, for example, flattened out physically for the image acquisition, the anatomical image can also be a 2D image. In a preferred embodiment, the anatomical image is a volume image of the coronary arteries of a heart of a patient.

The region of interest defining unit is configured to define a region of interest in the image for which the arterial feeding regions are to be determined. In particular, the region of interest can be any region of the anatomical image, even the whole image, and can be defined automatically, by a user or in a user machine interaction process. For example, the region of interest defining unit can be configured for utilizing known algorithms for roughly segmenting the main anatomical structures of the anatomical image and then outline the anatomical structures of interest as region of interest. However, the automatic defining of a region of interest can also be more simple, for instance, by simply defining a region at a specific, predetermined position of the anatomical image. Moreover, more sophisticated algorithms like machine learning algorithms that have been trained to identify and define a region of interest in an image can be utilized. If a user is to define the region of interest, the region of interest defining unit can be configured to present the anatomical image to a user, for instance, on a display and the user can then utilize input means, like a mouse, or a keyboard to indicate an outline of the region of interest for defining the region of interest. Moreover, in an interaction process the region of interest defining unit can be configured to provide the anatomical image to a user on which a region of interest is defined automatically, wherein in this case the user can then accept or amend the respective region of interest. It is noted here that it has been found that a rather rough definition of the region of interest is suitable for this method. In particular, even if the region of interest refers to the whole image, the method can be performed with suitable accuracy.

The geodesic distance map generation unit is configured to generate a geodesic distance map for the region of interest based on the anatomical image. In particular, a voxel of the geodesic distance map is given by a cumulated path cost from a predetermined seed to the respective voxel. It is noted that the formulation "a voxel given by" refers to determining the value of the voxel such that this expression can also be formulated as "a value of a voxel of the geodesic distance map is given by a cumulated path cost from a predetermined seed to the respective voxel". Preferably, the cumulated path cost for a voxel from a predetermined seed to the respective voxel is determined by calculating a path cost of all possible predecessor voxels, i.e. of all voxels in the neighborhood of the respective voxel that have already been assigned to a path, and to determine the least costly predecessor voxel. Generally, the path costs of possible predecessor voxels are already be known as calculated path cost value for the respective value, i.e. as a result of the calculation of the respective path cost for the respective possible predecessor voxels. Preferably, the cumulated path cost of the respective voxel is then determined based on the path cost for the least costly predecessor voxel added to a step cost for the path from the predecessor voxel to the respective voxel, wherein the step cost is calculated using a cost function. Thus, it is preferred that the cumulated path cost is determined such that each voxel only has one predecessor voxel with respect to a path but can comprise a plurality of successive voxels with respect to the path. Thus, from the predetermined seed a path can be split downwards into a plurality of paths, but each voxel can only be connected to one seed.

Generally, a path cost refers to a value that associates with a certain path or a part of a certain path from a predetermined starting point, i.e. a seed, to a respective voxel a certain value that is determined based on the cost function. In most cases, when determining a path cost, the absolute value of the path cost is not relevant, but only the relation of the cost of one path with respect to another path contains the relevant information, for instance, the information on the arterial feeding regions. However, if it is necessary, the cost function can be adapted such that also the absolute values of the path costs can be utilized, for example, by normalizing, weighting or otherwise adapting the respective cost function. Preferably, the geodesic distance map is generated utilizing a priority queue based front propagation method starting from the predetermined seed. Generally, it is possible to utilize not only one seed but a plurality of seeds, for instance, two or more seeds, and to calculate the respective geodesic distance map for each of the different seeds. In particular, this can have the advantage of providing further information on different arterial feeding regions. For example, the development of a respective path cost can indicate that the specific voxels belong to different arterial feeding regions, if the respective path cost is strongly dependent on the seed. Preferably, the seeds are set by a user, for instance, a medical practitioner, at or near an expected starting point of the arterial feeding system. However, the seeds can also be set automatically based on respective predetermined characteristics of the respective region of interest. Preferably, the seeds are determined automatically by utilizing a heuristic or machine-learning model. The model preferably detects the positions of arteries roots from which blood flows into the area of interest. For example, for a heart this can be the ostia-positions on the ascending aorta. However, if the model is not able to detect respective seed position, e.g. because of anatomical variants, it is preferred that the seeds are set manually using a graphical display.

The cost function refers to a function that takes into account a vesselness filter response and/or a contrast agent density determined, respectively, based on the image. Preferably, the cost function determines the path cost such that a high possibility of the voxel being part of a vessel and a good alignment between a current and a previous vessel direction estimation yields a low path cost, and vice versa. Moreover, if additionally or alternatively the contrast agent density is taken into account, it is preferred that a high contrast agent estimate in the region of the voxel leads to a low path cost in the cost function, and vice versa. Respective vesselness filters, in particular, local vesselness filters, which determine a local vesselness score for a voxel and optionally also a vesselness direction, are known. For example, a vesselness filter utilizing a Hessian matrix comprising second derivatives of a voxel can be utilized, or a vesselness filter utilizing a gradient with first derivatives of a voxel, or a vesselness filter utilizing a search-ray such as the Radial Structure Tensor, can be utilized. Generally, a vesselness filter response determines a score indicative of a probability that a voxel is part of a vessel in a medical image. The contrast agent density can be determined based on the image utilizing also respective known methods, for instance, determining the grayscale values of the voxel and/or one or more surrounding voxels in order to determine the amount of contrast agent indicated by the grayscale value. Since the contrast agent is present in the highest concentrations in the vessels, in particular, in the arteries, shown by the image, the contrast agent density is also indicative of the presence of a vessel at a position of a voxel. Thus, the cost function determines the path costs based on the probability of the presence of a vessel, in particular, an artery, in the region of a respective voxel for which the path cost should be determined. This allows the algorithm to follow the arterial vessels until the blood is distributed into the tissue and thus to clearly indicate which tissue region is fed by which artery. In a preferred embodiment, both the vesselness filter response and the contrast agent density are utilized. Further, the cost function can also take additional features into account, for example, if a segmentation of the vessels for the region of interest is available, the cost function can further utilize the segmentation of the vessels for determining the path cost. Moreover, the cost function can also take an alignment of a path direction with a general direction of a vessel, for instance, an artery, into account. In particular, the alignment with the general direction of the vessel from the root to the vessel to its destined tissue, for example, from a root of the aorta to an apex of the left ventricle. Preferable, the direction refers to the flow direction of blood through the vessels. For this additional feature the path cost can be the lower the better the path direction follows the general direction of the vessel. Furthermore, the cost function can be configured to take a local vessel radius into account. For example, the local vessel radius can be determined from a segmentation, if available. Preferably, the local vessel radius is taken into account by taking a comparison of the local vessel radius with a general tapering-off of the vessel thickness into account. Such a tapering-off of the vessel thickness is typical towards the lower branches of a vessel, for instance, towards an apex of the ventricle. For example, the cost function can determine a higher cost for voxels the longer the path, since it becomes more and more unlikely that the voxel is still part of the path of a blood vessel.

In a preferred embodiment the cost function $f$ comprises the form of

$$f = d[1 + exp(-\alpha D_c + \beta V)],$$

wherein d refers to the voxel neighbour distance, $D_c$ refers to the contrast agent density, $V$ to the vesselness filter response and the coefficients $\alpha$ and $\beta$ to optimizable variables.

The geodesic distance map providing unit is configured for providing the geodesic distance map to a user and/or to further processing to determine the arterial feeding regions. Since the geodesic distance map comprises all information necessary for determining the arterial feeding regions, a user can determine the arterial feeding regions by simply viewing the geodesic distance map very easily. However, the geodesic distance map can also be processed further for determining the arterial feeding regions, for instance, automatically.

In a preferred embodiment, the apparatus further comprises a geodesic distance map processing unit for processing the geodesic distance map such that arterial feeding regions of the organ in the region of interest are determined based on the geodesic distance map, and a mapping unit for mapping the determined arterial feeding regions to a representation of the organ. In particular, the geodesic distance map providing unit can provide the geodesic distance map to the geodesic distance map processing unit. The geodesic distance map processing unit can be configured for processing the geodesic distance map to present the geodesic distance map to a user, wherein the user can then utilize respective input means for determining the arterial feeding regions of the organ in the region of interest, for example, by providing a contour, outline or overlay in the geodesic distance map that indicates the respective feeding regions. However, the geodesic distance map processing unit can also be configured for automatically processing the geodesic distance map. For example, respective rules and characteristics of arterial feeding regions can be stored and utilized to determine the arterial feeding regions based on the geodesic distance map. For example, based on determined paths to a respective tissue region provided by the geodesic distance map it can be determined which part of an artery feeds the respective region. Preferably, for determining a feeding region for a voxel of the geodesic distance map the steepest descent of the path costs is followed from the voxel to a respectively known and optionally labeled vessel, for instance, one of the hear arteries. A boarder between two feeding regions can also be determined by following the steepest descent of the path costs of two neighboring voxel of the geodesic distance map that are not connected via a path to a common predecessor, i.e. a voxel that belongs to the path of both voxels to a common seed. If the length difference of the path following the steepest descent to the common predecessor is more than a predetermined threshold, the neighboring voxel belong to different feeding regions and a border between feeding regions is determined between the neighboring voxels.

The mapping unit is then configured to map the determined arterial feeding regions to a representation of the organ, for instance, by providing an overlay, a visible contour, a color indication, etc. to an image or atlas representation of the organ. The such determined map can then be presented as arterial feeding map to a user and can assist the user in the diagnosis and treatment of a respective patient.

In an embodiment, the apparatus further comprises an optimization unit for optimizing the cost function by comparing the generated geodesic distance map with the contrast agent distribution in the anatomical image, wherein based on the comparison the cost function is optimized and a geodesic distance map is generated based on the optimized cost function. Generally, the cost function can be optimized by optimizing one or more variables of the cost function. The variables of the cost functions can refer, for instance, to weights utilized for weighting different influences in the cost function on the path cost, for example, the influences of the vesselness filter response and the contrast agent density on the result of the path cost calculation. However, the variables can also refer to other quantities influencing the result of the cost function that are not determined, for example, by the anatomical image. Generally, such variables can be predetermined, for instance, based on an experience of a user, but it is preferred to adapt these variables to each case individually in order to increase the accuracy of the respective resulting geodesic distance map. However, the cost function can also be optimized by changing the influence features of the cost function, i.e. by changing the cost function itself. For example, instead of using only the vesselness filter response as influence feature, the contrast agent density can be used additionally or alternatively in a changed cost function. Also, for example, a kind of vesselness filter utilized for determining the vesselness filter response could be changed. This allows for example to also optimize the cost function such that the most influential influence feature is taken into account by the cost function.

Since the contrast agent distribution is a directly measurable variable, i.e. is part of the anatomical image provided and indicates the blood flow through the vessels in the region of interest which should be mapped by the geodesic distance map, an optimization of the cost function based on the comparison between the geodesic distance map and the contrast agent distribution in the anatomical image allows to adapt the cost function to each patient individually and thus to provide more accurate geodesic distance maps. The optimization can be performed utilizing any known optimization method, for instance, an iterative optimization method in which one or more iterative steps are performed until a difference between the generated geodesic distance map and the contrast agent distribution is minimized with respect to the respectively used cost function.

Preferably, the comparison is based on determining a correlation metric between the geodesic distance map and the contrast agent density in the anatomical image. Utilizing a correlation metric that is indicative of a correlation between the geodesic distance map and the contrast agent density has the advantage that the absolute values of the geodesic distance map and the contrast agent density are less relevant in the comparison than the relative values and thus the structure of the respective geodesic distance map and the contrast agent density. This allows for a fast and accurate comparison between the geodesic distance map and the contrast agent density in the anatomical image. Preferably, the correlation metric utilizes the absolute magnitude of a cross-correlation and/or a cross entropy between the geodesic distance map and the contrast agent density in the anatomical image.

In an embodiment, the cost function is optimized with respect to one or more variables that are linear weights of quantities of the cost function and wherein the optimization of the one or more variables is based on solving a linear system of equations based on the cost function for each voxel of the geodesic distance map. For example, a cost function can comprise a predetermined number of influences, for example, the vesselness filter response and/or the contrast agent density, wherein each of these influences comprises at least one weighted quantity, in particular, the influence itself can be the quantity that is linearly weighted. The path cost for a voxel is generally the sum of all previous voxel path costs on the respective path of the voxel that also respectively depend on a respectively predetermined number of weights M. For N voxels, this leads thus to a linear equation system of N equations with M unknown weights. Such a system is overdetermined and can be solved with known methods, for instance, with linear algebra methods.

In an embodiment, the cost function is derived as a regression value from a voxel neighborhood for each voxel. Preferably, in this case, the cost function depends linearly on a neighborhood of a respective voxel from which the regression value is calculated, for instance, the cost function can comprise a matrix multiplication of K weights with the neighborhood of the voxel. Then, a predetermined number N of voxels in the geodesic distance map can be utilized to form a linear equation system of n equations for the K unknown weights of the cost function, wherein also this linear equation system can then also be solved with known methods, in particular, with linear algebra. However, alternatively, the regression can be determined utilizing machine learning regression algorithms, like neural networks. In this case the machine learning regression algorithms can be trained for determining the path cost of a voxel based on the anatomical image values of the neighboring voxels. A training data set can comprise respective anatomical images, and thus anatomical image values of neighboring voxels, and respectively desired path costs, i.e. geodesic distance maps, for the respective voxels. The machine learning regression algorithms can then be parameterized based on the training data set.

In an embodiment, the optimization is based on determining a mismatch error as a difference in the compared geodesic distance map and the contrast agent density and back propagating the mismatched error along the tree-graph structure of the geodesic distance map to a respective cost function in order to optimize the cost function. In this case, directly a relative mismatch error, i.e. a difference between a voxel in a geodesic distance map and a contrast agent density, can be determined. Since it is known that the mismatch error has to be caused by the calculated path costs of the path of the respective voxel, for example, the variables utilized for determining the respective path cost in the cost function can be amended until the mismatch error is minimized. This direct optimization of the cost function is possible since each voxel only belongs to one path such that it is clear that the respective mismatch in the geodesic distance map and the contrast agent density of the voxel can only be caused by the respective cost function for this one path.

In an embodiment, the image providing unit is configured to provide an additional image, wherein the additional image is an anatomical image acquired after the administration of a contrast agent under different life parameters of the patient, wherein the geodesic distance map generation unit is configured to generate an additional geodesic distance map based on the additional image and wherein the apparatus further comprises an optimization unit configured to compare the geodesic distance map with the additional geodesic distance map for optimizing the cost function. Generally, different life parameters of a patient can refer to any life parameters that are suitable for causing a difference in the vessels, in particular, in the arteries of the patient. For example, different life parameters of a patient can be caused by a different stress status of the patient like a rest status versus an exerted status. Further, different pharmacological means can be utilized to initiate such different life parameters of the patient, for instance, by administering vessel-influencing drugs.

In an embodiment, the image providing unit is configured for providing as image a dynamic time series of images acquired during the propagation of the contrast agent through the tissue of the organ of the patient, wherein the geodesic distance map generation unit is configured to a) generate a geodesic distance map based on at least two of the images of the dynamic time series, respectively, resulting in at least two geodesic distance maps and b) generate a geodesic time-curve for at least one point of interest based on the at least two geodesic distance maps and a contrast agent time-curve for the at least one point of interest based on the dynamic time series of images, wherein the apparatus further comprises an optimization unit configured to correlate the geodesic time-curve and the contrast agent time-curve for optimizing the cost function. Preferably, a geodesic distance map is generated for each image that is part of the dynamic time series of images acquired during the propagation of the contrast agent to the tissue of the organ of the patient. This allows for a more accurate time curve. Generally, the time curve is indicative of the flow of the contrast agent through a respective part of the body of the patient represented by a voxel in a respective image. Thus, the geodesic time curve provides this information based on the geodesic distance maps and the contrast agent time curve provides this information directly based on the measured contrast agent density that is provided by the respective images of the dynamic time series. The relative course of the time curves, i.e. the geodesic time curve, and the contrast agent time curve for a voxel, should thus be the same. Respective correlation methods as described, for instance, above, can then be utilized to determine the correlation between the geodesic time curve and the contrast agent time curve to determine the difference and an optimization algorithm, for instance, an iterative algorithm, can be utilized to minimize the difference between the contrast agent time curve and the geodesic time curve with respect to the cost function. In particular, an overall correlation of a plurality of geodesic time curves and contrast agent time curves for a plurality of selected voxels, i.e. points of interest, can be used as objective function for the optimization of the cost function.

In a further aspect of the invention, a method is presented for assisting in determining a functional subdivision into arterial feeding regions in an organ of a subject, wherein the method comprises a) providing an image of an organ of a subject, wherein the image is an anatomical image acquired after the administration of a contrast agent, b) defining a region of interest in the image for which the arterial feeding regions are to be determined, c) generating a geodesic distance map for the region of interest based on the image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image, d) processing the geodesic map such that arterial feeding regions of the organ in the region of interest are determined based on the geodesic distance map, and e) mapping the determined arterial feeding regions to a representation of the organ. The method is a computer implemented method that can be realized on any computer hardware utilizing a respective software product..

In a further aspect of the invention, a system is presented for assisting in determining a functional subdivision into arterial feeding regions in an organ of a subject, wherein the system comprises a) an image acquisition unit for acquiring an image of an organ of a subject after the administration of a contrast agent, and b) an apparatus as described above.

In a further aspect of the invention, a computer program product is presented for assisting in determining a functional subdivision into arterial feeding regions in an organ of a subject, wherein the computer program product causes an apparatus as described above to carry out the method as described above.

It shall be understood that the apparatus as described above, the system as described above, the method as described above and the computer program product as described above have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplary a system for assisting in determining a functional subdivision into arterial feeding regions in an organ of a subject,
Fig. 2 shows schematically and exemplary a flowchart of a method for assisting in determining a functional subdivision into arterial feeding regions in an organ of the subject,
Fig. 3 shows an exemplary illustration of the determination of an anatomical region of interest,
Fig. 4 shows exemplary an illustration of a development of a geodesic distance map based on one seed, and
Fig. 5 shows exemplary an illustration of the determination of arterial feeding regions and the development of a geodesic distance map.

DETAILED DESCRIPTION OF EMBODIMENTS

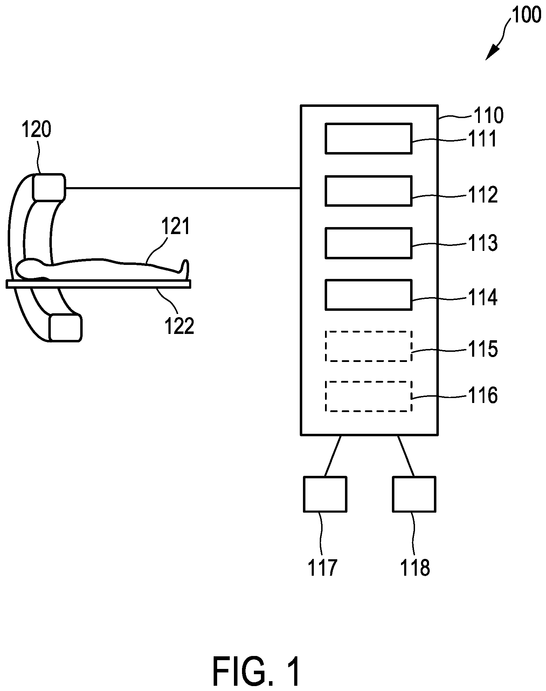

Fig. 1 shows schematically and exemplarily a system for assisting in determining a functional subdivision into arterial feeding regions in an organ of a subject. The system comprises an imaging acquisition unit 120 configured to acquire an image of a patient 121 lying on a patient table 122. Generally, the acquired image is an anatomical image acquired after the administration of a contrast agent. The image acquisition unit 120 can be, for instance, a CT system that allows to acquire a three-dimensional CT image of an anatomical structure of the patient 121. In a preferred embodiment, the anatomical structure of the patient 121 is a heart of the patient. Generally, for the anatomical structure being some kind of organ of the patient, the arterial feeding regions are to be determined, for instance, for diagnostic or treatment purposes.

The system 100 comprises an apparatus 110 for assisting in determining a functional subdivision into arterial feeding regions in an organ of a subject, for instance, of patient 121. The apparatus 110 therefore comprises an image providing unit 111 that is configured to provide an image of the organ of the subject, for instance, of patient 121. For example, the image providing unit 111 can be communicatively coupled with the image acquisition unit 120 for directly receiving the anatomical image of the patient 121 and providing the same. However, the image acquisition unit 120 can also first store the acquired image on a storage, for instance, a cloud storage, and the image providing unit 111 can then be configured for accessing the storage and receiving the image from the storage for providing the same.

The region of interest definition unit 112 is configured for determining a region of interest in the image for which the arterial feeding regions are to be determined. For example, the region of interest definition unit 121 can present the image to a user utilizing an output unit like a display 117, wherein the user can then utilize an input unit, for instance, a mouse or a keyboard 118, for defining the region of interest. For example, a contour of the region of interest can be drawn by the user on an image including the region of interest. However, the region of interest definition unit 112 can also be configured to automatically define a region of interest, for instance, based on predetermined criteria. For example, the region of interest definition unit 112 can be configured for segmenting anatomical structures within the image and can be provided with a predetermined rule that determines that a segmented part of the heart is a region of interest or any other part of a segmented anatomical structure. Generally, the region of interest can also be the whole image, wherein in this case the region of interest definition unit can simply determine that the whole image is the region of interest.

The geodesic distance map generation unit 113 of the apparatus 110 is then configured to generate a geodesic distance map for the region of interest based on the anatomical image. Generally, a value of a voxel of the geodesic distance map is given by a cumulated path cost from a predetermined seed to the respective voxel. For example, the geodesic distance map generation unit 113 can present the image to a user, for instance, via display 117 such that the user can indicate one or more seeds from which the geodesic distance map is to be determined. This allows a user to influence via the determination of the seeds the generation of the geodesic distance map. However, the one or more predetermined seeds can also be set automatically, for instance, by the geodesic distance map generation unit 113, for instance, based on predetermined rules, a predetermined position, predetermined characteristics of the image, a segmentation of the image, etc. Starting from the one or more predetermined seeds, for example, a priority queue based front propagation method can then be utilized for calculating for respective voxels at the front of the front propagation the cumulated path cost. For calculating a cumulated path cost, it is preferably determined for a respective voxel which neighboring voxel being part of a path comprises a least highest path cost. This voxel can then be regarded as the predecessor of the respective voxel along a path to which the respective voxel belongs. The cumulated path cost is then calculated by utilizing the path cost to the path through the predecessor voxel plus the path cost to the respective voxel from the predecessor voxel. For example, since the path cost for the respective predecessor voxel is already known, i.e. is provided by the value of the voxel, the respective value of the predecessor voxel can simply be added to a further path cost determined for the path between the predecessor voxel and the respective voxel.

If a priority queue based front propagation method is utilized for the calculation of the geodesic distance map, the calculation of the voxels follows the following algorithm. First one or more seeds are submitted as first entry into a waiting-queue for initialization of the algorithm. Then, the propagation of the front is always iterated by extracting the waiting entry with the currently lowest cost from the waiting-queue, i.e. the voxel at the front with the lowest path cost of all voxels in the waiting-queue at the front. This entry, i.e. voxel, is then processed by searching for so far unqueued and unprocessed neighbor voxels, i.e. voxel for which so far no path cost has been determined, for each of which its cost is determined, for instance as described above, as the sum of the current voxel, i.e. parent node and the respectively currently added additional step for the potential predecessor voxel, and entered into the waiting-queue. Thus, the front and accordingly the determination of the geodesic map in this embodiment propagates the fastest along the path with the least costs, i.e. along the expected path the blood takes from the arteries into the tissue. The path propagation algorithm can be stopped when either the queue is empty, i.e. for all voxels a path cost has been determined, or when the next calculated path cost exceed a certain threshold, e.g. when all possible feeding paths have been followed into the tissue, or the computation exceeds a certain predetermined computation time or computer memory constraint.

The path cost between voxels along a path is determined based on a cost function taking into account a vesselness filter response and/or a contrast agent density. Preferably, the cost function takes both features into account that can be determined based on the image. Utilizing a vesselness filter response and/or a contrast agent density for calculating a cost function allows to increase the past cost for voxels which are not part of a vessel, in particular, are not part of an artery, but, for instance, part of a tissue and to decrease the path cost for voxels that have a high likelihood for being part of a vessel, for instance, an artery. This allows during the propagation of the front for generating the geodesic distance map to identify the path along which a fluid, in particular blood, travels and dissipates into the tissue.

The geodesic distance map providing unit 114 can then provide the geodesic distance map to a user, for instance, by utilizing display 117. A user can then easily distinguish based on the geodesic distance map the different arterial feeding regions in an organ of the subject. However, a user can then also, for instance, if the arterial feeding regions are not yet clear, input one or more further seeds and restart the generation of the geodesic distance map based on the amended one or more seeds. However, the geodesic distance map providing unit 114 can also provide the determined geodesic distance map to the optional geodesic distance map processing unit 115. The geodesic distance map processing unit 115 is then configured for processing the geodesic distance map, in particular to determine the arterial feeding regions of the organ in the region of interest based on the geodesic distance map. For example, in this case the user can directly indicate, for instance, by utilizing a contour, the arterial feeding regions in the geodesic distance map. However, the geodesic distance map processing unit 115 can also be configured to automatically determine arterial feeding regions of the organ in the region of interest based on one or more characteristics of the respective geodesic distance map. An optional mapping unit 116 can then be configured for mapping the determined arterial feeding regions to a representation of the organ, for instance, to the anatomical image and/or any other representation of the organ like a contour representation, a segmented part of the organ, an atlas representation of the organ, etc. Based on this map, a user can directly view the arterial feeding regions in the organ and base further diagnostics and treatment options on this knowledge. In particular, utilizing the apparatus 110, the arterial feeding regions in an organ can be determined very accurately for each individual patient.

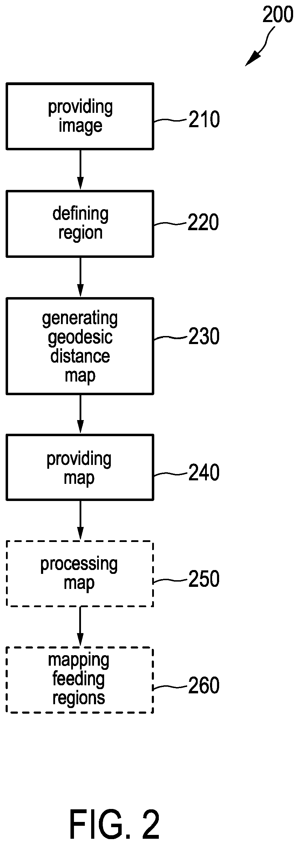

Fig. 2 shows schematically and exemplarily a flowchart of a method for assisting in determining a functional subdivision into arterial feeding regions in an organ of a subject. Generally, the method 200 can be performed by and in accordance with the principles described with respect to Fig. 1 and apparatus 110. In a first step 210, the method comprises providing an image of an organ of a subject, wherein the image is an anatomical image acquired after the administration of a contrast agent. In a next step 220, a region of interest is defined in the image for which the arterial feeding regions are to be determined. Further, the method 200 comprises a step 230 of generating a geodesic distance map for the region of interest based on the anatomical image in accordance with the principles described with respect to Fig. 1 and apparatus 110. In step 240, the geodesic distance map can then be provided to a user or alternatively for further processing to step 250 in which the geodesic distance map is processed such that arterial feeding regions of the organ in the region of interest are determined based on the geodesic distance map. In further optional step 260 the determined arterial feeding regions can then be mapped to a representation of the organ.

In the following, preferred embodiments and examples are described in more detail. Generally, Spectral CT assessment of the cardiac region has advantages over conventional, in particular, non-spectral, CT because of its superior contrast agent sensitivity. It is thus beneficial for functional assessment of the heart muscle and its feeding coronary arteries. Contrast agent uptake in vascular, in particular, coronary arteries, as well as muscular regions, in particular, myocardium, can serve as a surrogate for perfusion, even though small tissue-embedded vessels have calibers below the spatial resolution limit of the scanner. Perfusion in the myocardial areas is functionally related to the feeding coronary arteries, so that regional relation between coronary lesions and myocardial perfusion defects is of diagnostic value. For determining in this context arterial feeding regions, a schematic atlas of the myocardium is widely used, e.g. AHA segments, that is schematic, geometrically defined and not patient-specific. Normally, the atlas is a rigid mapping into 17 coarse areas and does not utilize functional image information to indicate blood-supply feeding territories.

The insight leading to invention, as already described above, for example, with respect to Figs. 1 and 2, is that an observed contrast agent, for instance, iodine, distribution in the arteries and in the tissue, e.g. in both coronary arteries as well as in the myocardium, can be regarded as a surrogate for vascular and tissue perfusion and can be 'explained' by a tree-graph structure rooted in the arterial supply and the respective vessel network, e.g. the coronary network. Utilizing this insight allows to provide a functional subdivision of an organ, e.g. the myocardium, into separate arterial feeding, i.e. perfusion territories, based on a single , optionally static, given image after administration of a contrast agent. Moreover, it can be considered that a computationally low-cost and thus fast determination of a propagation path along highly contrasted and vessel-like lines/regions in an anatomical image correlates with a dissipation of an arterial blood supply to areas of high perfusion, and the dissipation paths can thus be considered to correlate to functional feeding territories.

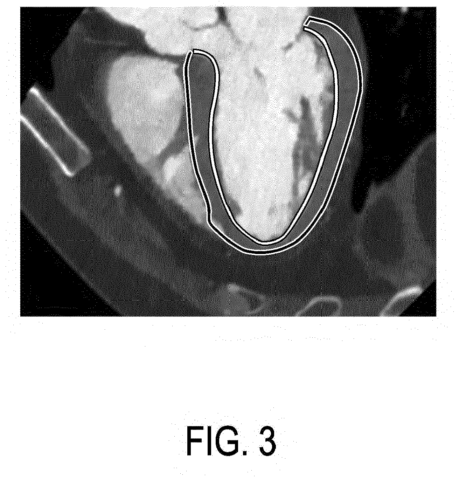

In the following details of a preferred example of the invention applied to determining the feeding regions in a myocardium of a heart of the patient are described. In a first step of the preferred method that can be performed, for example, by the region of interest defining unit a region of interest is defined in the organ, e.g. a heart, shown by an anatomical image. In particular, a cardiac region-of-interest model can be provided in order to define the region of interest in the image. For example, in a representation of a mesh model, or a labeled volume image yielding a labeling, i.e. semantic segmentation, of the anatomical structures of interest of the heart, the respective region of interest can be defined and transferred to the anatomical image. In this example, the myocardium surrounding the left or right cardiac ventricle, as well the ostia as the roots of the coronary tree can be defined as region of interest, as shown, for example, in Fig. 3.

In a following step the geodesic distance map is calculated for the defined region of interest based on the anatomical image. For example, from one or more seeds, i.e. tree roots, a geodesic distance map is computed using a local cost function. Each voxel of the geodesic map is given by the cumulated path cost of the seed to the point represented by the voxel. The identity of the seed, i.e. a branch label, to which the path to a respective voxel belongs can then be associated with the voxel and is inherited from the path predecessor of the voxel, which is the neighbor voxel with the lowest cumulated path cost, i.e. distance, so far. As each voxel of the geodesic map has one defined predecessor path voxel, an implicit tree-graph structure is constructed. Optionally, variables utilized in the cost function can further be optimized. For example, by an adaptation of the geodesic distance map to the observed contrast appearance by optimization of the local cost function, i.e. the variables of the local cost function. Examples and possibilities for such an optimization are described further below. Generally, the resulting geodesic map depends on the local cost function preferably such that decreasing costs are determined for increasing contrast agent density and increasing vesselness filter response. The goodness of fit of the geodesic distance map can then be evaluated by correlation with the contrast agent density map, for example, with the anatomical image being a spectral CT image, as a perfusion surrogate. The local cost function, which can be a global function with local arguments, can then be varied until an optimal correlation between the geodesic distance map and the observed contrast agent pattern is achieved. The variation can be the choice of the function, or a variation of variables, for example, function coefficients, while the function arguments are given by the observed image volume of interest. In a further step, for instance, performed by the geodesic distance map processing unit, the graphs provided by the geodesic distance map can be divided into sub-trees, For example, in case only a single seed, e.g., in the aortic bulbus, was provided, or a finer division is desired than the number of provided seeds, the tree-graph on which the geodesic distance map is based can be divided into subtrees, for example, using dendrogram splitting techniques. The new path labels and, if applicable, new path costs are then re-propagated to the voxels of the geodesic distance map. Moreover, the geodesic distance map processing unit can further be configured for the mapping of perfusion territories, i.e. feeding regions, determined based on a processing of the geodesic distance map to graphical representations of the organ. For example, resulting perfusion territories can be visualized graphically as borders, or mapped using a pseudo-color scheme. The mapping can be applied to graphical representations such as 3D renderings, schematic 2D bulls-eye-views, flattened manifolds, i.e. 'worldmaps', scrollable slice-wise CT viewports, etc.

Generally the main advantages of the invention as exemplarily described above, are that patient-specific functional perfusion territories instead of schematic atlas territories can be presented to a user as they are derived from a specific patient image, for instance CT scan in a rest and stress phase. Moreover, a presentation of the vascular flow in the coronaries can be an intrinsic part of the muscular area representation. Further, the described invention is particularly well suited to exploit the added benefits of spectral CT information, for example, lowering the cost function along paths with increasing contrast agent density. Additionally, the method is algorithmically concrete and tractable, and can be computed in typical interactive time spans. Furthermore, the method does not require a prior explicit segmentation of the coronaries, which may be prone to errors.

In the following some further possible optional embodiments are described. In an embodiment, the geodesic distance map and respective tree-graph of the geodesic distance map can be calculated from one or more seeds, e.g. positioned at an ostia provided by the anatomical model. Moreover a priority-queue based front propagation method can be utilized.

In an embodiment, the tree-graph used for calculating the geodesic distance map can be split into feeding areas. For example, based on the computed geodesic distance map and its implicit tree-graph, the split into principal branches, indicating feeding areas, can be done, e.g. by interactive user selection, or automatically, e.g. by using a dendrogram in conjunction with rule-based or atlas-based schemes, or by unsupervised clustering techniques.

Further, in the following some embodiments for the optimization of the cost function are described in more detail that can be performed by the optional optimization unit. In an embodiment a goodness-of-fit metric can be utilized for the optimization. For example, for the optional adaptation, i.e. optimization, of the cost function such that the geodesic distance map fits to the observed perfusion appearance, i.e. contrast agent image, a metric can be utilized to compare the computed geodesic distance map with the observed contrast agent image from a spectral-CT. A low geodesic path cost is, for example, expected to coincide with a high perfusion. An appropriate comparison metric is an absolute magnitude of cross-correlation, or the cross entropy between the computed geodesic distance map and measured, i.e. observed contrast agent density values in the anatomical region of interest, e.g. coronaries and myocardium. After the optimization the best achieved good-of-fit can serve as a confidence measure, i.e., if the correlation between geodesic map and observed iodine pattern is not sufficient, then the underlying assumption is violated, and a warning is issued to the user.

Generally, the local cost function can be considered as an inverse to a local speed function, and is a globally valid function, but its local cost function value depends on local properties, such as contrast agent density, and magnitude, orientation and width estimate from an appropriate vesselness filter, etc. A very simple parametric cost function could e.g. be $f = d[1 + exp(-\alpha D_c + \beta V)]$, wherein $D$ refers to the voxel neighbour distance, $D_c$ refers to the contrast agent density, $V$ to the vesselness filter response and the coefficients $\alpha$ and $\beta$ to optimizable variables. The function choice or the function coefficients can then be varied in order to optimize the goodness-of-fit.

In an embodiment variables of the cost function, for example, diffusion parameters, can be fitted by a general optimization. For example, if the local cost function contains only very few variables, general numerical optimization schemes can be applied to optimize the correlation between contrast agent density and geodesic distance map in the volume of interest.

In an embodiment, variables of the cost function, for instance, diffusion parameters, can be optimized by a feature weights regression. For example, in case of linear weights of the local cost function, a linear system of equations for M unknowns can be provided from the N voxels in the geodesic distance map in the volume of interest, which can be solved in closed-form. With the updated function weights, in this case the variables, the geodesic distance map can be recomputed for a number of iterations until convergence.

In an embodiment variables of the cost function, for instance, diffusion parameters, can be optimized by a feature weights back propagation. Since the geodesic distance map implicitly provides a tree-graph structure, a mismatch error of a final graph nodes, i.e. voxel values, can be propagated back to each local cost function, which can then be changed tentatively, until convergence.

In an embodiment, variables of the cost function, for instance, diffusion parameters, can be optimized by a general non-linear regression. It is also possible to use a non-parametric local cost function, derived as a regression value from neighbor voxel values of a local, e.g. 9x9x9, voxel neighborhood of a voxel. The regression can be trained based on respective training samples. Generally, there is one training sample for each voxel and its path-predecessor voxel of the distance map, wherein the training sample is a collection of neighboring image voxel values of the voxel, for instance, determined based on the anatomical image, and a desired path cost, i.e. the desired difference between the path cost of the voxel and its predecessor. As vector-input regressors, for example, neural networks (NN), support vector machines (SVM), or Random Forests (RF), can be utilized.

In an embodiment, the optimization can be based on two phase images. In this case, two phase image volumes are provided acquired with different patient live parameters, e.g. when the patient is stressed an relaxed. Then the optimization of the local cost function can be performed as to satisfy the regions of interest in both images simultaneously. The spatial consistency of the two computed geodesic maps can then be used as another optimization and confidence criterion.

In an embodiment, the optimization can be based on dynamic time-series scans. In case not only a single static spectral CT scan is available, but rather a dynamic time series of scan volumes, then for each location of the volume of interest a bolus curve of the contrast agent can be established and correlated with the 'propagation' time, i.e. the cost value, of the geodesic distance map. This can be used for further optimization of the local cost function, e.g. by modelling the relation between vessel diameter and blood flow, and the relation between Hounsfield density and blood flow optionally considering e.g. the incompressibility of fluid and calcifications.

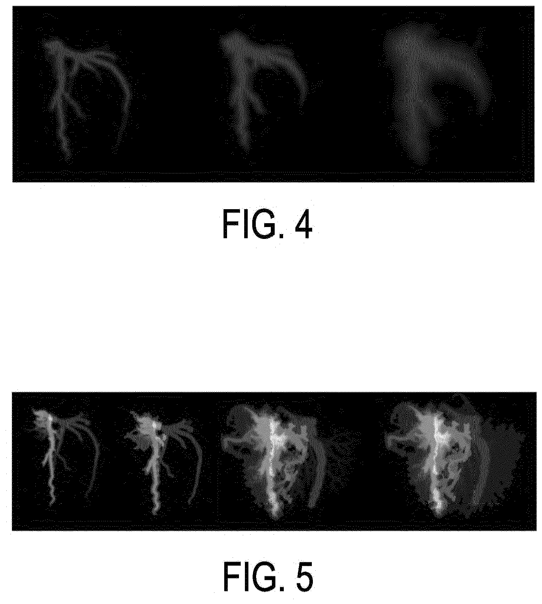

Fig. 4 shows exemplary an illustration of a development of a geodesic distance map based on one seed. In particular, Fig. 4 shows the evolving front propagation of the geodesic distance map from a seed, i.e. root point in the top left corner of the image using a local cost function with decreasing cost at high contrast agent density and high vesselness response.

Fig. 5 shows exemplary an illustration of the determination of arterial feeding regions and the development of a geodesic distance map. In particular, Fig. 5 shows two subtree labels, i.e. a geodesic distance map generated based on two seed, being propagated together with the evolving geodesic distance map, defining the influence areas as indication of anatomical perfusion feeding territories. It is noted here that this illustration is only a concept illustration and contains, for instance, a mis-tracing at a vessel crossing.

Although, the above embodiments are mainly described with respect to cardiac spectral CT, the invention can also be applied in an analogous fashion to conventional CT, cardiac MR, or other cardiac modalities, like ultrasound, in which perfusion is manifesting by virtue of contrast agent uptake. Moreover, the invention can also be applied to organs other than the heart with supplying vessels, in particular to a lung or liver.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of an anatomical image, the defining of a region of interest, the generating of a geodesic distance map, the providing of the geodesic distance map, etc. performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

It is an object of the invention to provide an apparatus that allows for assisting a user in determining an improved subdivision into arterial feeding regions in an organ of a patient. An image providing unit provides an image of an organ of a subject. A geodesic distance map generation unit generates a geodesic distance map for a region of interest based on the anatomical image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image. A geodesic distance map providing unit provides the geodesic distance map to a user and/or to further processing to determine the arterial feeding regions.

**Claims**

1. An apparatus for assisting in determining a functional subdivision into arterial feeding regions in an organ of a subject (121), wherein the apparatus (110) comprises:

   - an image providing unit (111) for providing an image of an organ of a subject (121), wherein the image is an anatomical image acquired after the administration of a contrast agent,
   - a region of interest defining unit (112) for defining a region of interest in the image for which the arterial feeding regions are to be determined,
   - a geodesic distance map generation unit (113) for generating a geodesic distance map for the region of interest based on the anatomical image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image,
   - a geodesic distance map providing unit (114) for providing the geodesic distance map to a user and/or to further processing to determine the arterial feeding regions.

2. The apparatus according to claim 1, wherein the apparatus (110) further comprises a geodesic distance map processing unit (115) for processing the geodesic distance map such that arterial feeding regions of the organ in the region of interest are determined based on the geodesic distance map, and a mapping unit (116) for mapping the determined arterial feeding regions to a representation of the organ.

3. The apparatus according to any of claims 1 and 2, wherein the apparatus (110) further comprises an optimization unit for optimizing the cost function by comparing the generated geodesic distance map with the contrast agent distribution in the anatomical image, wherein based on the comparison the cost function is optimized and a geodesic distance map is generated based on the optimized cost function.

4. The apparatus according to claim 3, wherein the comparison is based on determining a correlation metric between the geodesic distance map and the contrast agent density in the anatomical image.

5. The apparatus according to claim 4, wherein the correlation metric utilizes the absolute magnitude of a cross-correlation and/or a cross entropy between the geodesic distance map and the contrast agent density in the anatomical image.

6. The apparatus according to any of claims 3 to 5, wherein the cost function is optimized with respect to one or more variables that are linear weights of quantities of the cost function and wherein the optimization of the one or more variables is based on solving a linear system of equations based on the cost function for each voxel of the geodesic distance map.

7. The apparatus according to any of claims 2 to 5, wherein the cost function is derived as a regression value from a voxel neighborhood for each voxel.

8. The apparatus according to any of claims 3 to 5, wherein the optimization is based on determining a mismatch error as a difference in the compared geodesic distance map and the contrast agent density and back propagating the mismatched error along the tree-graph structure of the geodesic distance map to a respective cost function in order to optimize the cost function.

9. The apparatus according to any of the preceding claims, wherein the image providing unit (111) is configured to provide an additional image, wherein the additional image is an anatomical image acquired after the administration of a contrast agent under different life parameters of the patient, wherein the geodesic distance map generation unit (113) is configured to generate an additional geodesic distance map based on the additional image and wherein the apparatus (110) further comprises an optimization unit configured to compare the geodesic distance map with the additional geodesic distance map for optimizing the cost function.

10. The apparatus according to any of the preceding claims, wherein the image providing unit (111) is configured for providing as image a dynamic time series of images acquired during the propagation of the contrast agent through the tissue of the organ of the patient, wherein the geodesic distance map generation unit (113) is configured to a) generate a geodesic distance map based on at least two of the images of the dynamic time series, respectively, resulting in at least two geodesic distance maps and b) generate a geodesic time-curve for at least one point of interest based on the at least two geodesic distance maps and a contrast agent time-curve for the at least one point of interest based on the dynamic time series of images, wherein the apparatus (110) further comprises an optimization unit configured to correlate the geodesic time-curve and the contrast agent time-curve for optimizing the cost function.

11. The apparatus according to any of the preceding claims, wherein the cost function f comprises the form of

$$f = d[1 + exp(-\alpha D_c + \beta V)],$$

wherein $D$ refers to the voxel neighbour distance, $D_c$ refers to the contrast agent density, $V$ to the vesselness filter response and the coefficients $\alpha$ and $\beta$ to optimizable variables..

12. The apparatus according to any of the preceding claims, wherein the geodesic distance map is generated utilizing a priority queue based front propagation method starting from the predetermined seed.

13. A method for assisting in determining a functional subdivision into arterial feeding regions in an organ of a subject (121), wherein the method (200) comprises:

- providing (210) an image of an organ of a subject (121), wherein the image is an anatomical image acquired after the administration of a contrast agent,
- defining (220) a region of interest in the image for which the arterial feeding regions are to be determined,
- generating (230) a geodesic distance map for the region of interest based on the anatomical image, wherein a voxel of the geodesic distance map is given by a cumulated path cost from a predetermined seed to the respective voxel and wherein the path cost is determined based on a cost function taking into account a vesselness filter response and/or a contrast agent density determined, respectively, based on the image,
- providing (240) the geodesic distance map to a user and/or to further processing to determine the arterial feeding regions.

14. A system for assisting in determining a functional subdivision into arterial feeding regions in an organ of a subject, wherein the system (100) comprises:

   - an image acquisition unit (120) for acquiring an image of an organ of a subject after the administration of a contrast agent,
   - an apparatus (110) according to any of claims 1 to 12.

15. A computer program product for assisting in determining a functional subdivision into arterial feeding regions in an organ of a subject (121), wherein the computer program product causes an apparatus (110) according to any of claims 1 to 12 to carry out the method (200) according to claim 13.

FIG. 1

200

```
┌─────────────────┐
│    providing    │——210
│      image      │
└─────────────────┘
         │
         ▼
┌─────────────────┐
│    defining     │——220
│     region      │
└─────────────────┘
         │
         ▼
┌─────────────────┐
│   generating    │
│    geodesic     │——230
│    distance     │
│      map        │
└─────────────────┘
         │
         ▼
┌─────────────────┐
│    providing    │——240
│       map       │
└─────────────────┘
         │
         ▼
┌─ ─ ─ ─ ─ ─ ─ ─ ─┐
│   processing    │——250
│       map       │
└─ ─ ─ ─ ─ ─ ─ ─ ─┘
         │
         ▼
┌─ ─ ─ ─ ─ ─ ─ ─ ─┐
│    mapping      │
│    feeding      │——260
│    regions      │
└─ ─ ─ ─ ─ ─ ─ ─ ─┘
```

# FIG. 2

FIG. 3

FIG. 4

FIG. 5

Europäisches
Patentamt

European
Patent Office

Office européen
des brevets

# EUROPEAN SEARCH REPORT

Application Number

EP 22 21 2260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Termeer Maurice ET AL: "Patient-Specific Mappings between Myocardial and Coronary Anatomy", , 1 January 2010 (2010-01-01), XP093015557, DOI: 10.4230/dfu.sciviz.2010.196 Retrieved from the Internet: URL:chrome-extension://efaidnbmnnnibpcajpc glclefindmkaj/https://drops.dagstuhl.de/op us/volltexte/2010/2705/pdf/14.pdf [retrieved on 2023-01-19] | 1-5,8, 13,15 | INV. G06T7/11 G06T7/187 |
| Y | * Section 5 * <br> * Section 1 * <br> * Section 3 * <br> * figure 2 * <br> ───── | 6 | |
| L | LORENZ C ET AL: "Simultaneous segmentation and tree reconstruction of the coronary arteries in MSCT images", PROCEEDINGS OF SPIE, IEEE, US, vol. 5031, 1 January 2003 (2003-01-01), pages 167-177, XP002325790, DOI: 10.1117/12.480314 ISBN: 978-1-62841-730-2 * Section 1 * * Section 2 * <br> ───── | 1-6,8, 13,15 | |
|  | -/-- | | TECHNICAL FIELDS SEARCHED (IPC) <br><br> G06T |

The present search report has been drawn up for all claims

| Place of search | Date of completion of the search | Examiner |
|---|---|---|
| Munich | 8 February 2023 | Yilmaz, Özgün |

EPO FORM 1503 03.82 (P04C01)

page 1 of 5

Europäisches Patentamt

European Patent Office

Office européen des brevets

## EUROPEAN SEARCH REPORT

Application Number

EP 22 21 2260

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BELIVEAU P ET AL: "Computation of coronary perfusion territories from CT angiography", COMPUTERS IN CARDIOLOGY, 2007, IEEE, PISCATAWAY, NJ, USA, 30 September 2007 (2007-09-30), pages 753-756, XP031404821, ISBN: 978-1-4244-2533-4 * abstract * * Section 2 * * figure 1 * * figure 3 * * figure 2 * | 1,2, 13-15 | |
| X | HORTENSE A KIRIÅ LI ET AL: "Comprehensive visualization of multimodal cardiac imaging data for assessment of coronary artery disease: first clinical results of the SMARTVis tool", INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE, vol. 7, no. 4, 24 September 2011 (2011-09-24), pages 557-571, XP035082993, ISSN: 1861-6429, DOI: 10.1007/S11548-011-0657-2 | 1,2,7,9, 11-13,15 | |
| Y | * Section Patient-specific Coronary Territory Map * * Section CTCA image analysis * * figure 6 * | 6 | TECHNICAL FIELDS SEARCHED (IPC) |
| | -/-- | | |

The present search report has been drawn up for all claims

| Place of search | Date of completion of the search | Examiner |
|---|---|---|
| Munich | 8 February 2023 | Yilmaz, Özgün |

EPO FORM 1503 03.82 (P04C01)

page 2 of 5

Europäisches
Patentamt

European
Patent Office

Office européen
des brevets

# EUROPEAN SEARCH REPORT

**Application Number**

EP 22 21 2260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | METZ C ET AL: "Coronary centerline extraction from CT coronary angiography images using a minimum cost path approach", MEDICAL PHYSICS, AIP, MELVILLE, NY, US, vol. 36, no. 12, 9 November 2009 (2009-11-09), pages 5568-5579, XP012129830, ISSN: 0094-2405, DOI: 10.1118/1.3254077 * Section II.B * * figure 6 *  ----- | 6 | |
| X | FRIAS ANTONIO E. ET AL: "Using dynamic contrast-enhanced MRI to quantitatively characterize maternal vascular organization in the primate placenta : DCE-MRI of the Primate Placenta", MAGNETIC RESONANCE IN MEDICINE, vol. 73, no. 4, 18 April 2014 (2014-04-18), pages 1570-1578, XP093017277, US ISSN: 0740-3194, DOI: 10.1002/mrm.25264 Retrieved from the Internet: URL:https://api.wiley.com/onlinelibrary/tdm/v1/articles/10.1002%2Fmrm.25264> * Section Summary Purpose * * Section Segmentation of Arrival Time Maps * * Section Analysis DCE-MRI * * Section Determination of Volumetric Flow for Each Perfusion Unit * * Section Summary Methods * * figure 4 * * figure 3 * * figure 6 * * Section Methods *  -----  -/-- | 1,2,10, 13-15 | **TECHNICAL FIELDS SEARCHED (IPC)** |

The present search report has been drawn up for all claims

| Place of search | Date of completion of the search | Examiner |
|---|---|---|
| Munich | 8 February 2023 | Yilmaz, Özgün |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**page 3 of 5**

Europäisches
Patentamt
European
Patent Office
Office européen
des brevets

**EUROPEAN SEARCH REPORT**

Application Number

EP 22 21 2260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIRISLI H A ET AL: "A patient-specific visualization tool for comprehensive analysis of coronary CTA and perfusion MRI data", MEDICAL IMAGING 2011: VISUALIZATION, IMAGE-GUIDED PROCEDURES, AND MODELING, SPIE, 1000 20TH ST. BELLINGHAM WA 98225-6705 USA, vol. 7964, no. 1, 3 March 2011 (2011-03-03), pages 1-9, XP060008089, DOI: 10.1117/12.873050 [retrieved on 2011-03-01] * the whole document * | 1-15 | |
| A | HERNANDEZ-VELA A ET AL: "Accurate Coronary Centerline Extraction, Caliber Estimation, and Catheter Detection in Angiographies", IEEE TRANSACTIONS ON INFORMATION TECHNOLOGY IN BIOMEDICINE, IEEE SERVICE CENTER, LOS ALAMITOS, CA, US, vol. 16, no. 6, 1 November 2012 (2012-11-01), pages 1332-1340, XP011490995, ISSN: 1089-7771, DOI: 10.1109/TITB.2012.2220781 * the whole document * | 1-15 | TECHNICAL FIELDS SEARCHED (IPC) |
| A | MABROUK S ET AL: "Multiscale Graph Cuts Based Method for Coronary Artery Segmentation in Angiograms", IRBM, ELSEVIER, AMSTERDAM, NL, vol. 38, no. 3, 15 May 2017 (2017-05-15), pages 167-175, XP085076141, ISSN: 1959-0318, DOI: 10.1016/J.IRBM.2017.04.004 * the whole document * | 1-15 | |

-/--

The present search report has been drawn up for all claims

| Place of search | Date of completion of the search | Examiner |
|---|---|---|
| Munich | 8 February 2023 | Yilmaz, Özgün |

EPO FORM 1503 03.82 (P04C01)

page 4 of 5

Europäisches
Patentamt

European
Patent Office

Office européen
des brevets

# EUROPEAN SEARCH REPORT

Application Number

EP 22 21 2260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | K Krissian ET AL: "Minimally Interactive Knowledge-based Coronary Tracking in CTA using a Minimal Cost Path", Midas Journal, 8 July 2008 (2008-07-08), XP055687482, https://www.midasjournal.org/browse/publication/593 Retrieved from the Internet: URL:http://hdl.handle.net/10380/1435 [retrieved on 2020-04-20] * the whole document * | 1-15 | |
| A | MAURICE ALAIN TERMEER: "Comprehensive Visualization of Cardiac MRI Data", INTERNET CITATION, 1 December 2008 (2008-12-01), page 135pp, XP007906953, Retrieved from the Internet: URL:http://www.cg.tuwien.ac.at/research/publications/2009/termeer-2009-cvc/termeer-2009-cvc-thesis.pdf [retrieved on 2009-01-27] * the whole document * | 1-15 | |
| A | JANG YEONGGUL ET AL: "Geodesic Distance Algorithm for Extracting the Ascending Aorta from 3D CT Images", COMPUTATIONAL AND MATHEMATICAL METHODS IN MEDICINE, vol. 2016, 1 January 2016 (2016-01-01), pages 1-7, XP093015045, ISSN: 1748-670X, DOI: 10.1155/2016/4561979 Retrieved from the Internet: URL:http://downloads.hindawi.com/journals/cmmm/2016/4561979.pdf> * the whole document * | 1-15 | TECHNICAL FIELDS SEARCHED (IPC) |

The present search report has been drawn up for all claims

| Place of search | Date of completion of the search | Examiner |
|---|---|---|
| Munich | 8 February 2023 | Yilmaz, Özgün |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)